# EUROPEAN PATENT SPECIFICATION

(11) **EP 2 120 991 B1**
(45) Date of publication and mention of the grant of the patent: **29.01.2014**
(21) Application number: 08712603.3
(22) Date of filing: 11.02.2008
(51) Int. Cl.: A61K 38/07, A61P 7/04, A61K 38/04, A61K 38/24

(54) **TREATMENT OF TRAUMA HEMORRHAGE WITH SHORT OLIGOPEPTIDES**
BEHANDLUNG VON TRAUMATISCHER HÄMORRHAGIE MIT KURZEN OLIGOPEPTIDEN
TRAITEMENT DE L'HÉMORRAGIE TRAUMATIQUE AVEC DES OLIGOPEPTIDES COURTS

(30) Priority: 12.02.2007 US 901155 P; 23.07.2007 US 961841 P
(43) Date of publication of application: 25.11.2009
(73) Proprietor: Biotempt B.V., 2514 EN Den Haag (NL)
(72) Inventor: KHAN, Nisar, Ahmed, NL-3039 JL Rotterdam (NL); BENNER, Robbert, NL-2992 SH Barendrecht (NL)
(74) Representative: Hatzmann, Martin
(86) International application number: PCT/NL2008/050077
(87) International publication number: WO 2008/100140

(56) References cited:
- EP-A- 1 466 612
- WO-A-01/11048
- WO-A-96/33218
- ALTAVILLA DOMENICA ET AL: "Tumour necrosis factor-alpha as a target of melanocortins in haemorrhagic shock, in the anaesthetized rat" BRITISH JOURNAL OF PHARMACOLOGY, vol. 124, no. 8, August 1998 (1998-08), pages 1587-1590, XP002484272 ISSN: 0007-1188

## Description

### TECHNICAL FIELD

The invention relates generally to biotechnology and medicine.

### BACKGROUND

Injury is the fifth leading cause of death worldwide and will become the second leading cause by 2020. It is already the leading cause of death in individuals aged 5 to 45 yeats. Vehicular injury, self inflicted injury, interpersonal violence (including war), work-related injury, falls, burns, and environmental disasters all contribute their share.

Primary prevention is the most effective way to limit injury. The importance of education, engineering controls, and the rule of law in prevention of injury cannot be overemphasized. Almost as many individuals die of vehicular injury in Egypt as in the U.S., but Egypt has one-quarter the population and one-tenth the number of vehicles. Nevertheless, the majority of injuries in both countries are preventable.

Secondary prevention, the application of acute care to prevent death and disability following injury, is also highly effective. The cost of trauma care is low per quality-adjusted life year saved compared with treatments in other common disease categories such as cardiovascular illness, stroke, or cancer interventional therapy. This care is best provided in regional centers. The largest Level 1 trauma centers typically see more than 5000 direct admissions each year and are staffed around the clock with trauma surgeons, neurosurgeons, orthopedists, anesthesiologists, and a complete array of support staff. The work of these centers in patient care, medical education, and developing new knowledge is driving an international revolution in the quality of injury care.

Every year, 1 in 7 Americans is significantly injured. Two-thirds of those, or 1 person in 10 of the U.S. population, seek medical care for that injury, and 1 out of every 100 Americans is admitted to a hospital for injury care each year. About 1 in every 10 patients admitted for injury, or 1 out of every 1000 Americans, receives blood products in the course of injury care. These individuals receive 10% to 15% of all of the blood transfused in the U.S.

Severe hemorrhage and hemorrhagic shock are common causes of morbidity and mortality in critically ill patients in intensive care. Patients in shock have impaired macro- and microcirculation in various tissue beds. Impaired splanchnic perfusion plays an important role in the development of multiple organ dysfunction owing to enhanced bacterial translocation from the gut and activation of an exacerbated inflammatory cascade. Decreased splanchnic perfusion also leads to the low blood supply to the downstream organs, such as the liver, leading to hepatic dysfunction, which also contributes to multiple organ failure after shock.

About 156,000 people die of injury each year in the U.S., and 93,000 of those fatalities involve physical trauma. Half of these individuals die before they reach the hospital. Among those who reach the hospital alive and who will die during that hospital admission, 80% die within the first 24 hours after admission. The most frequent causes of death of patients who die in the field or in the hospital are profound neurologic injury and uncontrolled hemorrhage.

Control of hemorrhage is a critical aspect of trauma care. In the field, bandages, direct pressure, and tourniquets control superficial and extremity hemorrhage. In the hospital, diagnostic imaging and surgical exploration allow the rapid identification of most other sites of bleeding. However, identification of sites of injury does not always allow immediate control of hemorrhage. Injuries such as deep hepatic lacerations and pelvic fractures with disruption of the pelvic venous plexus frequently require packing, and control of bleeding is obtained only slowly. These injuries can result in extensive and prolonged bleeding even in the hospital.

Patterns of blood use following traumatic injury are determined by the patterns of injury, the speed of transport to surgical care, and the availability of resources at the surgical center. At the University of Maryland R. Adams Cowley Shock Trauma Center in Baltimore in calendar year 2000, 91% of 5649 patients admitted directly from the scene of injury received no blood products. About two-thirds of the remainder, 332 patients, received 10 U of red blood cells (RBCs) or less. However, 75% of the RBCs administered were given to the 146 patients who received more than 10 U and 50% of all the RBCs used were administered to 68 patients, who received more than 20 U of RBCs each. Thus, a select group of trauma patients receive transfusion, and it is at these patients who are changing our thinking about blood use and resuscitation and towards treatment of whom this invention is directed.

Criteria decisive for the decision to resuscitate or transfuse a patient suspected to undergo trauma-hemorrhage are diverse and complex, (see for example Critical Care, Management of Bleeding Following Major Trauma: a European Guideline, Posted 04/02/2007, Donat R. Spahn; Vladimir Cerny; Timothy J. Coats; Jacques Duranteau; Enrique Fernandez-Mondéjar; Giovanni Gordini; Philip F. Stahel; Beverley J. Hunt; Radko Komadina; Edmund Neugebauer; Yves Ozier; Louis Riddez; Arthur Schultz; Jean-Louis Vincent; Rolf Rossaint, http://www.medscape.com/viewarticle/554058 1 and on) however, at a certain moment it is decided whether and how to resuscitate by providing such patients with RBC's or plasma, platelets or other bloodproducts

### SUMMARY OF THE INVENTION

Herein we determine whether administration of short oligopeptides has any effect on deleterious immune functional parameters after trauma-hemorrhage.

Disclosed herein are methods and associate means for treating a subject (e.g., a mammal such as a human), experiencing, diagnosed as experiencing, or thought to be at risk for experiencing hemorrhagic shock. Such methods include administering, to the subject in a medically or pharmaceutically acceptable manner, a short oligopeptide such as AQGV and/or LQGV. Therewith the invention relates to use of at least one isolated or synthetic tetrapeptide selected from the group consisting of LQGV, AQGV and LAGV and functional derivatives thereof wherein an L-amino acid residue is substituted with a D-amino acid, or a pharmaceutically acceptable acid- or base addition salt thereof, for the production of a pharmaceutical composition for the treatment of a subject suffering from or believed to be suffering from trauma-hemorrhage. Said peptide preferably identified by testing at least one isolated or synthetic peptide in an experimental animal model of trauma-hemorrhage and demonstrating that administration of said testpeptide after induction of trauma-hemorrhage reduces the plasma level of at least one pro-inflammatory cytokine (for example TNFalfa or IL-6 as provided herein) in an animal subjected to trauma-hemorrhage when compared with an animal subjected to trauma-hemorrhage that has not been provided with a testpeptide. Said peptide consists of 4 amino acids selected from the group consisting of AQGV, LQGV or LAGV. Treatment with mixtures of peptides is also provided, which comprise at least one peptide selected from the group of AQGV, LQGV or LAGV, and an other peptide. A preferred other peptide is selected from those capable of reducing pro-inflammatory cytokine levels in an animal model of trauma-hemorrhage or hemorrhagic shock, such as one provided herein. In a preferred embodiment of the invention said treatment of trauma-hemorrhage or hemorrhagic shock also comprises providing (resuscitating or transfusing) said subject with blood or blood products such as red blood cells (RBC's), platelets, plasma, or combinations thereof.

Also described is a method for identifying a peptide, or functional analogue or derivative thereof, for use in the production of a pharmaceutical composition for the treatment of a subject suffering from or believed to be suffering from trauma-hemorrhage comprising testing at least one isolated or synthetic peptide of smaller than 30 amino acids in an experimental animal model of trauma-hemorrhage and demonstrating that administration of said testpeptide after induction of trauma-hemorrhage reduces the plasma level of at least one pro-inflammatory cytokine in an animal subjected to trauma-hemorrhage when compared with an animal subjected to trauma-hemorrhage that has not been provided with a testpeptide. It is preferred to test the testpeptide in a method wherein said animal subjected to trauma-hemorrhage is also provided with blood or blood products such as red blood cells (RBC's), platelets, plasma, or combinations thereof. Also described is selecting said testpeptide capable of reducing the desired pro-inflammatory cytokine levels for use in the production of a pharmaceutical composition, in particular wherein said pharmaceutical composition is produced for the treatment of a subject suffering from or believed to be suffering from trauma-hemorrhage hemorrhagic shock.

In hemorrhagic shock there is massive blood loss, which cannot be compensated by the body without treatment. The primary treatment of hemorrhagic shock is to control bleeding and restore intravascular volume to improve tissue perfusion. This treatment induces an inflammatory response, which may culminate into a severe inflammatory response and finally multiple organ dysfunction syndrome (MODS) ^{[1,2,3]}. In addition, approximately 40 % of patients develop sepsis as a result of trauma-hemorrhage [3]. Sepsis and MODS are the leading causes of death in critically ill patients on the intensive care unit all over the world with mortality rates of about 50% [4,5].

The severe inflammatory response due to trauma-hemorrhage is characterised by increased expression of adhesion molecules, such as intracellular adhesion molecule-1 (ICAM-1) and vascular cell adhesion molecule-1 (VCAM-1), on sinusoidal endothelial cells and hepatocytes. Furthermore, increased levels of pro-inflammatory cytokines are found systemically and locally in liver, lungs and intestine [6, 7, 8, 9]. The pro-inflammatory cytokines produced are in particular tumour necrosis factor alpha (TNF-α), interleukin (IL)-1β and IL-6 [10, 11, 12]. These cytokines affect organ integrity/function directly, but also indirectly through secondary mediators, such as nitric oxide, thromboxanes, leukotrienes, platelet-activating factor, prostaglandins, and complement ^{[13, 14]}. TNF-α also causes the release of tissue-factor by endothelial cells leading to fibrin deposition and disseminated intravascular coagulation ^{[15, 16]}. Cells within the liver, mainly Kupffer cells, but also hepatocytes and sinusoidal endothelial cells, are considered as the main producers of these pro-inflammatory cytokines during hemorrhagic shock ^{[17]}.
A fine balance between vasodilators and vasoconstrictors maintains splanchnic perfusion. Increased systemic production of vasoconstrictors such as epinephrine, angiotensin II, endothelin, and thromboxane A₂ has been observed in experimental models of trauma-hemorrhage and sepsis. These vasoconstrictors not only contribute to the increased total peripheral resistance but also act on the splanchnic vessels and reduce their perfusion rate. The reduced production of vasodilators or the attenuated response of the splanchnic vessel to the vasodilators (endothelial dysfunction) is also observed after severe hemorrhagic shock. Both of these factors contribute to the circulatory disturbance. In addition, these effects induce intestinal hypoxia, reduce nutrient supply, increase production of oxygen free radicals, and increase neutrophil accumulation, leading to damage of the intestinal mucosal barrier and thereby resulting in increased bacterial translocation.

The last decade, researchers have focused on the modulation of the systemic inflammatory responses with therapeutic agents aiming at neutralizing the activity of cytokines, especiallty TNF-α ^{[18]}. Other researchers used therapeutic agents aiming at the inhibition of TNF-α production ^{[19]}. However, most of these therapeutic agents must be administered before the onset of hemorrhagic shock to achieve a therapeutic effect ^{[19]}. Clearly, this is almost impossible in a clinical trauma-hemorrhage setting. Therefore, therapies initiated after the onset of severe trauma-hemorrhage and aiming at reducing the production of pro-inflammatory cytokine are more relevant to prevent the events leading to MODS.

During pregnancy, the maternal immune system tolerates the fetus by reducing the cell-mediated immune response while retaining normal humoral immunity ^{[20]}. Also, clinical symptoms of cell-mediated autoimmune diseases regress in many patients during pregnancy ^{[20]}. The hormone human chorionic gonadotropin (hCG) is mainly secreted by placental syncytiocytotrophoblasts during pregnancy and has been shown to be immunoregulatory ^{[21, 22, 23]}. The β-subunit of hCG is degraded by specific proteolytic enzymes ^{[24]}. This can lead to the release of several oligopeptides consisting of four to seven amino acids which, because of their role in regulation of physiological processes, are considered regulatory ^{[25]}. We successfully demonstrated that synthetic oligopeptides can inhibit the acute inflammatory response, disease severity, and mortality in high-dose lipopolysaccharide induced systemic inflammatory response syndrome ^{[26]}. Considering these powerful regulating effects of synthetic oligopeptides on inflammation, we hypothesized that the administration of such regulatory oligopeptides after severe trauma-hemorrhage could inhibit the massive inflammatory response, associated with this condition. To this end, we used LQGV, which is part of the primary structure of loop two of the β-subunit of hCG, and two alanine replacement variants, namely AQGV and LAGV.

In this study we demonstrate that LQGV, AQGV and LAGV, administrated after the induction of hemorrhagic shock in rats, significantly reduced TNF-α and IL-6 plasma levels, which is associated with reduced TNF-α and IL-6 mRNA transcript levels in the liver. This indicates that LQGV, AQGV, LAGV have therapeutic potential with beneficial effects on systemic inflammation, thereby reducing organ integrity/function, which is associated with severe hemorrhagic shock.

### BRIEF DESCRIPTION OF THE DRAWINGS

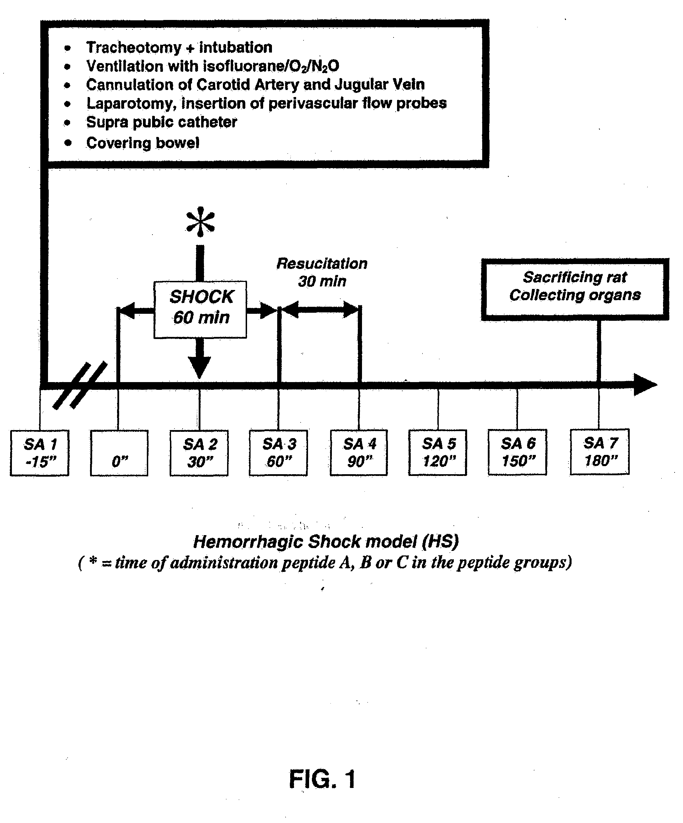

FIG. 1: Hemorrhagic Shock model (HS) (* = time of administration peptide A, B or C in the peptide groups).

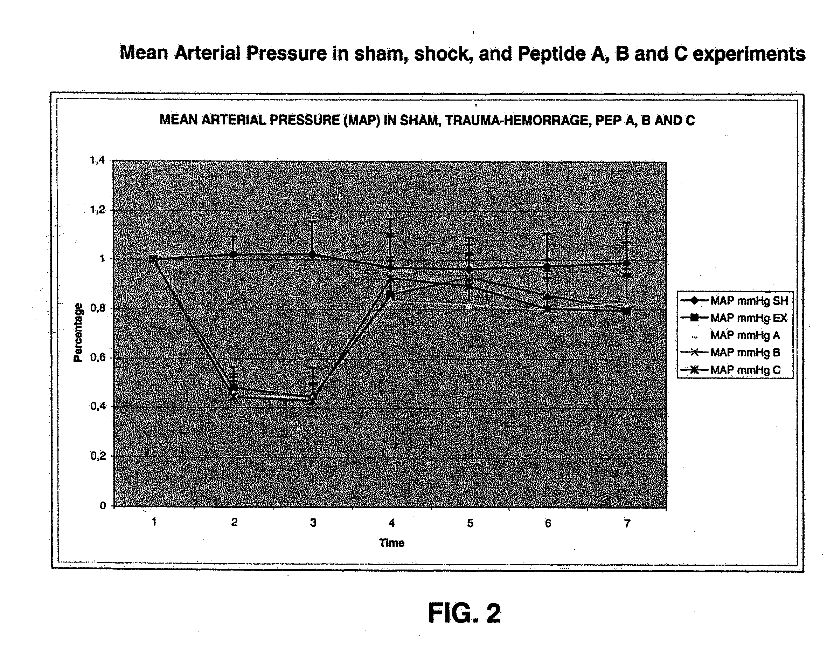

FIG. 2: Mean Arterial Pressure in sham, shock, and Peptide A, B and C experiment.

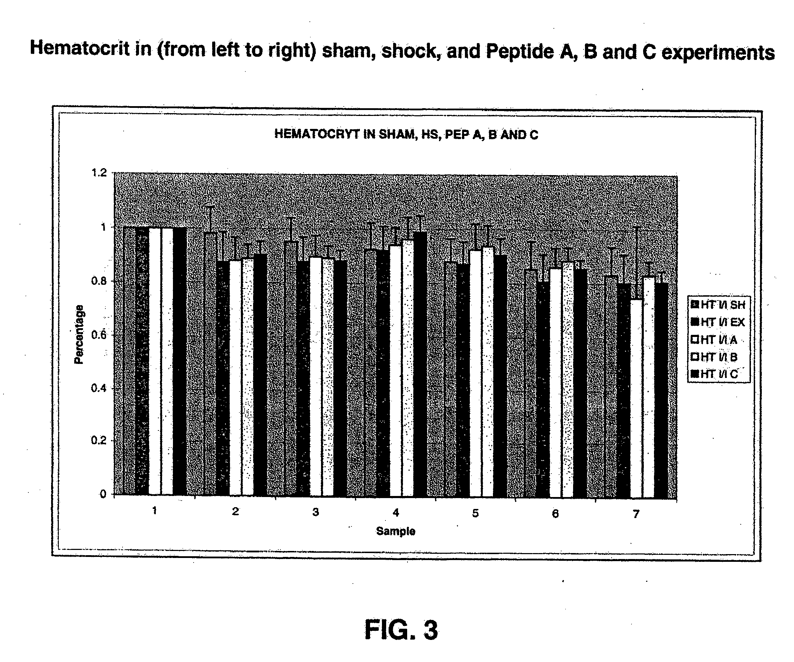

FIG. 3: Hematocrit in (from left to right) sham, shock, and Peptide A, B and C experiments.

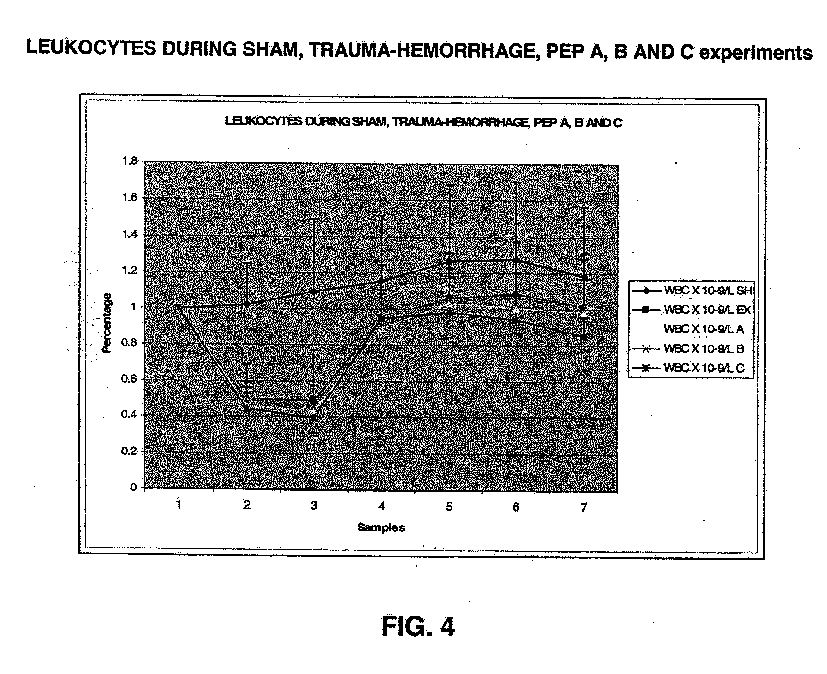

FIG. 4: Leukocytes during sham, trauma-hemorrhage, pep A, B and C experiments.

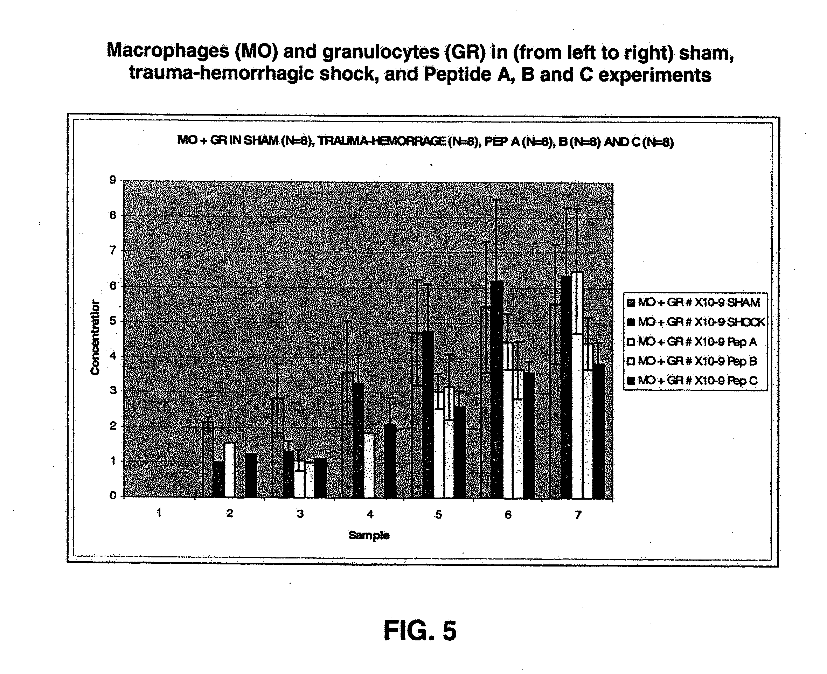

FIG. 5: Macrophages (MO) and granulocytes (GR) in (from left to right) sham, trauma-hemorrhagic shock, and Peptide A, B and C experiments.

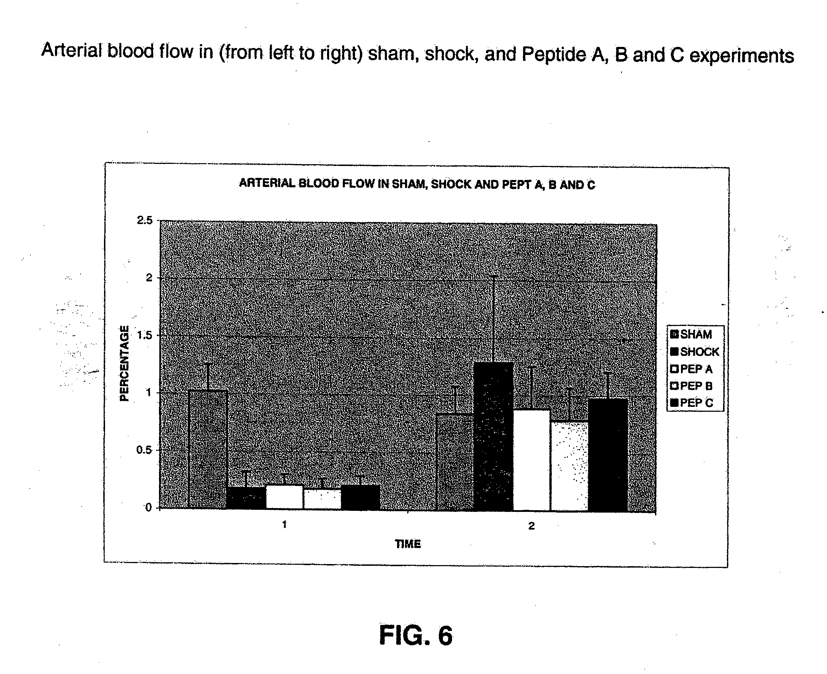

FIG. 6: Arterial blood flow in (from left to right) sham, shock, and Peptide A, B and C experiments.
FIG. 7. Hemorrhagic shock model. **A)** Schematic representation of the experimental design. **B)** The measured mmHg was recalculated in percentages to standardize the experiment and to compensate for animal differences. **C)** Percentage of leukocytes in blood during various time points of the experiment.
FIG. 8. TNF-α plasma levels in different experimental groups determined at 15 minutes before and 30, 60, 90, 120, 150 and 180 minutes after the onset of hemorrhagic shock. □ Sham, ○ HS, ∇ HS/LQGV, ◊ HS/AQGV, Δ HS/LAGV. Each figure represents one animal.
FIG. 9. IL-6 plasma levels in different experimental groups determined at 120, 150 and 180 minutes after the onset of hemorrhagic shock □ Sham, O HS, ∇ HS/LQGV, ◊ HS/AQGV, Δ HS/LAGV. Each figure represents one animal.
FIG. 10. Transcript levels for A) TNF-α, **B)** IL-6 and **C)** ICAM-1 in the liver, 180 minutes after the onset of hemorrhagic shock. Data expressed are correlated to GAPDH expression. □ Sham, ○ HS, ∇ HS/LQGV, ◊ HS/AQGV, Δ HS/LAGV. Each figure represents one animal

### DETAILED DESCRIPTION OF THE INVENTION

U.S. Patent 5,380,668 to Herron (Jan. 10, 1995), the contents of the entirety of which are incorporated by this reference, discloses, among other things, various compounds having the antigenic binding activity of hCG. Herron further discloses means and methods for making oligopeptides.

The compounds for use according to the general formula may be prepared in a manner conventional for such compounds. To that end, suitably N alpha protected (and side-chain protected if reactive side-chains are present) amino acid derivatives or peptides are activated and coupled to suitably carboxyl protected amino acid or peptide derivatives either in solution or on a solid support. Protection of the alpha-amino functions generally takes place by urethane functions such as the acid-labile tertiary-butyloxycarbonyl group ("Boc"), benzyloxycarbonyl ("Z") group and substituted analogs or the base-labile 9-fluoremyl-methyloxycarbonyl ("Fmoc") group. The Z group can also be removed by catalytic hydrogenation. Other suitable protecting groups include the Nps, Bmv, Bpoc, Aloc, MSC, etc. A good overview of amino protecting groups is given in The peptides. Analysis. Synthesis. Biology, Vol. 3 E. Gross and J. Meienhofer, eds. (Academic Press, New York, 1981). Protection of carboxyl groups can take place by ester formation, for example, base-labile esters like methyl or ethyl, acid labile esters like tert. butyl or, substituted, benzyl esters or hydrogenolytically. Protection of side-chain functions like those of lysine and glutamic or aspartic acid can take place using the aforementioned groups. Protection of thiol, and although not always required, of guanidino, alcohol and imidazole groups can take place using a variety of reagents such as those described in The Peptides, Analysis, Synthesis, Biology, *id*. or in Pure and Applied Chemistry, 59(3), 331-344 (1987). Activation of the carboxyl group, of the suitably protected amino acids or peptides can take place by the azide, mixed anhydride, active ester, or carbodiimide method especially with the addition of catalytic and racemization-suppressing compounds like 1-N--N-hydroxybenzotriazole, N-hydroxysuccinimide, 3-hydroxy-4-oxo-3,4-dihydro-1,2,3,-benzotria-zine, N-hydroxy-5 norbornene-2,3-dicar-boxyimide. Also the anhydrides of phosphorus based acids can be used. *See, e.g.,* The Peptides, Analysis, Synthesis, Biology, *supra* and Pure and Applied Chemistry, 59(3), 331-344 (1987).

It is also possible to prepare the compounds by the solid phase method of Merrifield. Different solid supports and different strategies are known, *see, e.g.,* Barany and Merrifield in The Peptides, Analysis, Synthesis, Biology, Vol. 2, E. Gross and J. Meienhofer, eds. (Acad. Press, New York, 1980), Kneib-Cordonier and Mullen Int. J. Peptide Protein Res., 30, 705-739 (1987) and Fields and Noble Int. J. Peptide Protein Res., 35, 161-214 (1990). The synthesis of compounds in which a peptide bond is replaced by an isostere, can, in general, be performed using the previously described protecting groups and activation procedures. Procedures to synthesize the modified isosteres are described in the literature, for instance, for the --CH₂-NH-- isostere and for the --CO--CH₂ -- isostere.

Removal of the protecting groups, and, in the case of solid phase peptide synthesis, the cleavage from the solid support, can take place in different ways, depending on the nature of those protecting groups and the type of linker to the solid support. Usually deprotection takes place under acidic conditions and in the presence of scavengers. *See, e.g.,* volumes 3, 5 and 9 of the series on The Peptides Analysis, Synthesis, Biology*, supra.*

Another possibility is the application of enzymes in synthesis of such compounds; for reviews see, e.g., H. D. Jakubke in The Peptides, Analysis, Synthesis, Biology, Vol. 9, S. Udenfriend and J. Meienhofer, eds. (Acad. Press, New York, 1987).

Although possibly not desirable from an economic point of view, oligopeptides according to the invention could also be made according to recombinant DNA methods. Such methods involve the preparation of the desired oligopeptide thereof by means of expressing recombinant polynucleotide sequence' that codes for one or more of the oligopeptides in question in a suitable microorganism as host. Generally the process involves introducing into a cloning vehicle (*e.g.*, a plasmid, phage DNA, or other DNA sequence able to replicate in a host cell) a DNA sequence coding for the particular oligopeptide or oligopeptides, introducing the cloning vehicle into a suitable eucaryotic or prokaryotic host cell, and culturing the host cell thus transformed. When a eucaryotic host cell is used, the compound may include a glycoprotein portion.

As used herein, a "functional analogue" or "derivative" of a peptide includes an amino acid sequence, or other sequence monomers, which has been altered such that the functional properties of the sequence are essentially the same in kind, not necessarily in amount. An analogue or derivative can be provided in many ways, for instance, through "conservative amino acid substitution." Also peptidomimetic compounds can be designed that functionally or structurally resemble the original peptide taken as the starting point but that are for example composed of non-naturally occurring amino acids or polyamides. With "conservative amino acid substitution," one amino acid residue is substituted with another residue with generally similar properties (size, hydrophobicity), such that the overall functioning is likely not to be seriously affected. However, it is often much more desirable to improve a specific function. A derivative can also be provided by systematically improving at least one desired property of an ammo acid sequence. This can, for unstance, be done by an Ala-scan and/or replacement net mapping method. With these methods, many different peptides are generated, based on an original ammo acid sequence but each containing a substitution of at least one amino acid residue. The amino acid residue may either be replaced by alanine (Ala-scan) or by any other ammo acid residue (replacement net mapping). This way, many positional variants of the original amino acid sequence are synthesized Every positional variant is screened for a specific activity. The generated data are used to design improved peptide derivatives of a certain amino acid sequence.

A derivative or analogue can also be, for instance, generated by substitution of an L-amino acid residue with a D-amino acid residue. This substitution, leading to a peptide that does not naturally occur in nature, can improve a property of an ammo acid sequence. It is, for example, useful to provide a peptide sequence of known activity of all D-amino acids in retro inversion format, thereby allowing for retained activity and increased half-life values. By generating many positional variants of an original amino acid sequence and screening for a specific activity, improved peptide derivatives comprising such D-amino acids can be designed with further improved characteristics.

A person skilled in the art is well able to generate analogous compounds of an ammo acid sequence. This can, for instance, be done through screening of a peptide library. Such an analogue has essentially the same functional properties of the sequence in kind, not necessarily in amount Also, peptides or analogues can be circularized, for example, by providing them with (terminal) cysteines, dimerized or multimerized, for example, by linkage to lysine or cysteme or other compounds with side-chains that allow linkage or multimerization, brought in tandem- or repeat-configuration, conjugated or otherwise linked to carriers known in the art, if only by a labile link that allows dissociation.

As used herein, an oligopeptide also includes, for example, an acceptable salt, base, or ester of the oligopeptide or a labeled oligopeptide. As used herein, "acceptable salt" refers to salts that retain the desired activity of the oligopeptide or equivalent compound, but preferably do not detrimentally affect the activity of the oligopeptide or other component of a system in which uses the oligopeptide. Examples of such salts are acid addition salts formed with inorganic acids, for example, hydrochloric acid, hydrobromic acid, sulfuric acid, phosphoric acid, nitric acid, and the like. Salts may also be formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, and the like. Salts may be formed with polyvalent metal cations such as zinc, calcium, bismuth, barium, magnesium, aluminium, copper, cobalt, nickel and the like or with an organic cation formed from N,N'-dibenzylethylenediamine or ethylenediamine, or combinations thereof (*e.g.*, a zinc tannate salt).

The oligopeptide, or its modification or derivative, can be administered as the entity, as such, or as a pharmaceutically acceptable acid- or base addition salt, formed by reaction with an inorganic acid (such as hydrochloric acid, hydrobromic acid, perchloric acid, nitric acid, thiocyanic acid, sulfuric acid, and phosphoric acid); or with an organic acid (such as formic acid, acetic acid, propionic acid, glycolic acid, lactic acid, pyruvic acid, oxalic acid, malonic acid, succinic acid, maleic acid, and fumaric acid); or by reaction with an inorganic base (such as sodium hydroxide, ammonium hydroxide, potassium hydroxide); or with an organic base (such as mono-, di-, trialkyl and aryl amines and substituted ethanolamines). A selected peptide and any of the derived entities may also be conjugated to sugars, lipids, other polypeptides, nucleic acids and PNA; and function in-situ as a conjugate or be released locally after reaching a targeted tissue or organ.

A pharmaceutical composition for use herein may be administered to the subject parenterally or orally. Such a pharmaceutical composition may consist essentially of (or consist of) oligopeptide and PBS. It is preferred that the oligopeptide is of synthetic origin. Suitable treatment, for example, entails administering the oligopeptide (or salt or ester) in the pharmaceutical composition to the patient intravenously in an amount of from about 0.0001 to about 35 mg/kg body mass of the subject. It may be useful that the pharmaceutical composition consists essentially of from one to three different oligopeptides.

The invention is further described with the aid of the following illustrative examples.

### EXAMPLES

### Example 1

### Materials and methods

Adult Male specific pathogen-free Wistar rats (Harlan CPB, Zeist, The Netherlands), weighing 350-400 g were used after a minimum seven-day acclimation period. The animals were housed under barrier conditions and kept at 25°C with a twelve-hour light/dark cycle. Rats were allowed free access to water and chow (-). All procedures were performed in accordance with the Principles of Laboratory Animal Care (NIH publication No. 86-23, revised 1985) under a protocol approved by the Committee on Animal Research of the Erasmus University (protocol EUR 365).

The rats were fasted overnight but were allowed free access to water before the experiment. Subsequent to endotracheal intubation the rats were mechanically ventilated with an isofluorane (-) N₂O/O₂ mixture at 60 breaths/minute. Body temperature was continuously maintained at 37.5°C by placing the animals on a thermo controlled "half-pipe" (UNO, The Netherlands). Polyethylene tubes (PE-50, Becton Dickinson; St. Michielsgestel, The Netherlands) were flushed with heparin and placed via the right carotid artery in the aorta and in the right internal jugular vein. The animals received no heparin before or during the experiment.

Mean arterial pressures (MAP) was measured using transducers (Becton Dickinson) that were connected in line to an electronic recorder (Hewlett Packard, 78354-A Germany) for electronically calculated mean pressures and continuous measurement of the animal's blood pressure. Under semi sterile conditions a median laparotomy was performed and ultrasonic perivascular flow probes (Transonic Systems Inc, Maastricht, The Netherlands) were placed on the common hepatic artery and the portal vein. A supra pubic catheter was placed to monitor the urine production during and after resuscitation.

After an acclimatization period of 20 minutes, the rats were randomized into the following five groups:

Hemorrhagic shock group were bled within ten minutes to a mean arterial pressure (MAP) of 40 mmHg and maintained at this level for 60 minutes by withdrawing or re-infusing shed blood as needed. Thereafter, the animals were resuscitated with plus minus four times the volume of the withdrawn blood over 30 minutes with a 0.9% NaCl solution.

The hemorrhagic shock group + peptide A (LAGV; one-letter amino acid code) underwent the same procedure as the hemorrhagic shock group but received a single bolus injection of 5 mg/kg peptide A intravenously 30 minutes after the induction of shock.

The hemorrhagic shock group + peptide B (AQGV) underwent the same procedure as the hemorrhagic shock group and received a single bolus injection of 5 mg/kg peptide B intravenously, 30 minutes after the induction of shock.

The hemorrhagic shock group + peptide C (LQGV) underwent the same procedure as the hemorrhagic shock group and received a single bolus injection of 5 mg/kg peptide C intravenously, 30 minutes after the induction of shock.

Sham group underwent the same procedure as the hemorrhagic shock group without performing the hemorrhage or administration of any kind of peptides.

The hepatic arterial blood flow (QHA) and hepatic portal venous blood flow (QVP) were measured with transit time ultrasonic perivascular flow probes, connected to an ultrasonic meter (T201; Transonic Systems, Inc., Maastricht, NL). Systemic and hepatic hemodynamics were continuously measured. At regular time points arterial blood samples were taken. The animals were euthanized by withdrawal of arterial blood via the carotid artery.

### Blood, tissue, and cell harvesting procedure

*Plasma collection and storage:* Whole arterial blood was obtained at -15, 30, 60, 90, 120, 150 and 180 minutes after induction of shock via the right carotid artery and collected in duplo. 0.2 ml was placed in tubes (Eppendorf EDTA KE/1.3) to be assayed in the coulter counter (-). 0.5 ml was placed in Minicollect tubes (Bio-one, Greiner) centrifuged for five minutes, immediately frozen, and stored at -80°C, until assayed. All assays were corrected for the hematocrit.

*Measurement of cytokines (still in progress):* The levels of IL-6, and IL-10 in the serum were determined by an ELISA (R&D Systems Europe Ltd) according to the manufacturer's instructions.

*Histology (still in progress):* The alterations in lung, liver, sigmoid and small bowel morphology were examined in sham-operated animals, in animals after trauma-hemorrhage and in animals after trauma-hemorrhage treated with peptide A, B or C. All tissues were collected in duplo. One part was harvested and fixed in formalin (Sigma) and later embedded in paraffin. The other part was placed in tubes (NUNC Cryo tube™ Vials), quick frozen in liquid nitrogen and stored at -80°C until assayed.

### Results

*Mean Arterial Pressure:* MAP dropped in all shock groups significant during the shock phase compared to the control group.

*Hematocrit:* The hematocrit following trauma-hemorrhage was similar in the different peptide A, B and C treated and non-treated groups. During the shock phase there was a difference of hematocrit in the control group in comparison with the other groups. From the resuscitation phase (90 minutes) there was no significant difference in hematocrit among the control, trauma-hemorrhage, and peptide groups.

*Leukocyte Recruitment:* During trauma-hemorrhage the leukocytes dropped from 100% at T0 in all groups to a minimum of 40.0 ± 11.9%, 42.0 ± 8.7%, 47.3 ± 12.4%, 38.2 ± 7.4% in respectively the non-treated, peptide A treated, peptide B treated and peptide C treated group because of leukocyte accumulation in the splanchnic microcirculation. There was a significant difference in leukocyte concentration between all treated and non-treated trauma-hemorrhage groups, and the control group during the shock phase. No significant difference was noticed between the peptide A, B or C treated animals and the non-treated animals.

*Blood Concentrations Of Macrophages And Granulocytes:* At 180 min after the onset of trauma-hemorrhage, concentrations of circulating macrophages (M_{Φ}) and granulocytes were significant lower in the peptide B and C treated animals compared with the corresponding experimental group. Blood levels of circulating M_{Φ} and granulocytes were 5,556 ± 1,698 10⁹/l in sham-operated animals whereas blood levels were 6,329 ± 1,965 10⁹/l after trauma-hemorrhage, and decreased by 29.9% after administration of peptide B (4,432 ± 0.736 10⁹/l) and 39.2% after administration of peptide C (3,846 ± 0.636 10⁹/l) compared with concentrations after trauma-hemorrhage.

*Arterial Hepatic Blood Flow:* There was a decrease in the arterial hepatic blood flow in the shock group (18.3 ± 14.3%) and in the peptide A (21.3 ± 9.1%), B (18.1 ± 9.0%) and C (21.2 ± 8.6%) group during the shock period compared with the control group (102.6 ± 23.5%). An increase in blood flow was observed during the reperfusion in the hepatic artery of the shock group (128.9 ± 75.4%) compared with control animals (83.7 ± 24.2%) and the animals treated with peptide B (78.4 ± 28.3%).

Trauma-hemorrhage results in hypoxic stress owing to the absolute reduction in circulating blood volume. In contrast, sepsis is an inflammatory state mainly mediated by bacterial products. It is interesting that these divergent insults reveal similar pathophysiologic alterations in terms of the splanchnic circulation.

Hemorrhagic shock significantly increases leukocyte accumulation in the splanchnic microcirculation owing to the up-regulation of P selectin. The expression of intercellular adhesion molecule within the intestinal muscular vasculature after hemorrhagic shock promotes the local recruitment of leukocytes, and this inflammatory response is accompanied by subsequent impairment of intestinal function.

The adhesion and extravasation of neutrophils not only contribute to the inflammatory response in the splanchnic tissue bed but also induce intestinal microcirculatory failure and dysfunction after severe stress. This is mediated by the induced expression of adhesion molecules, such as selectins and endothelial cell adhesion molecules, on the surface of neutrophils and endothelial cells.

In our shock experiments, leukocyte concentration significant decreases during hemorrhagic shock compared to the control animals. However a single dose of peptide B or C administered during resuscitation, decreased concentrations of circulating macrophages and granolocytes 120 minutes after the onset of hemorrhagic shock compared to the non-treated animals.

Because some female sex hormones effectively protect the organs from circulatory failure after various adverse circulatory conditions, numerous studies have been performed to clarify the molecular mechanism of for example estradiol action with regard to tissue circulation. In this study, a single dose of peptide was administered following trauma-hemorrhage and various parameters were measured at 3 hours following the induction of shock. Treatment with peptides improved or restored immune functional parameters and cardiovascular functions. Therefore, our results show that administration of short oligopeptides (NMPFs) is beneficial in the treatment of critically ill trauma victims experiencing hemorrhagic shock.

### Example 2

BACKGROUND: Hemorrhagic shock followed by resuscitation induces a massive pro-inflammatory response, which may culminate into severe inflammatory response syndrome, multiple organ failure and finally death. Treatments aimed at inhibiting the effects of pro-inflammatory cytokines are only effective when initiated before the onset of hemorrhagic shock, which severely limits their clinical application.

AIM: We investigated whether the administration of synthetic oligopeptides (LQGV, AQGV, LAGV) 30 minutes after induction of hemorrhagic shock reduced the inflammatory response.

METHODS: Rats were bled to 50% of baseline mean arterial pressure and one hour later resuscitated by autologous blood transfusion. Thirty minutes after onset of hemorrhagic shock, experimental groups received either one of the synthetic oligopeptides (LQGV, AQGV, LAGV) or 0.9% NaCl solution. TNF-α and IL-6 plasma levels were determined at fixed time points before and after onset of hemorrhagic shock. Liver, lungs, ileum and sigmoid mRNA levels for TNF-a, IL-6 and ICAM-1 were determined 180 minutes after onset of hemorrhage.

RESULTS: Treatment with either one of the three oligopeptides (LQGV, AQGV, LAGV) efficiently reduced TNF-α and IL-6 plasma levels as well as TNF-α and IL-6 mRNA transcript levels in the liver.

CONCLUSION: Considering these powerful effects of oligopeptides during severe hemorrhagic shock, they may have therapeutic potential with beneficial effects on the hyper inflammation, thereby reducing the late life threatening tissue- and organ-damage that is associated with severe hemorrhagic shock.

INTRODUCTION: In hemorrhagic shock there is massive blood loss, which cannot be compensated by the body without treatment. The primary treatment of hemorrhagic shock is to control bleeding and restore intravascular volume to improve tissue perfusion. This treatment induces an inflammatory response, which may culminate into a severe inflammatory response and finally multiple organ dysfunction syndrome (MODS) ^{[1, 2, 3]}. In addition, approximately 40 % of patients develop sepsis as a result of trauma-hemorrhage ^{[3]}. Sepsis and MODS are the leading causes of death in critically ill patients on the intensive care unit all over the world with mortality rates of about 50% ^{[4, 5]}.

The severe inflammatory response due to trauma-hemorrhage is characterised by increased expression of adhesion molecules, such as intracellular adhesion molecule-1 (ICAM-1) and vascular cell adhesion molecule-1 (VCAM-1), on sinusoidal endothelial cells and hepatocytes. Furthermore, increased levels of pro-inflammatory cytokines are found systemically and locally in liver, lungs and intestine ^{[6, 7, 8, 9]}. The pro-inflammatory cytokines produced are in particular tumour necrosis factor alpha (TNF-α), interleukin (IL)-1β and IL-6 ^{[10, 11, 12]}. These cytokines affect organ integrity/function directly, but also indirectly through secondary mediators, such as nitric oxide, thromboxanes, leukotrienes, platelet-activating factor, prostaglandins, and complement ^{[13, 14]}. TNF-α also causes the release or tissue-factor by endothelial cells leading to fibrin deposition and disseminated intravascular coagulation ^{[15, 16]}. Cells within the liver, mainly Kupffer cells, but also hepatocytes and sinusoidal endothelial cells, are considered as the main producers of these pro-inflammatory cytokines during hemorrhagic shock ^{[17]}.

The last decade, researchers have focused on the modulation of the systemic inflammatory responses with therapeutic agents aiming at neutralizing the activity of cytokines, especiallty TNF-α ^{[18]}. Other researchers used therapeutic agents aiming at the inhibition of TNF-α production ^{[19]}. However, most of these therapeutic agents must be administered before the onset of hemorrhagic shock to achieve a therapeutic effect ^{[19]}. Clearly, this is almost impossible in a clinical trauma-hemorrhage setting. Therefore, therapies initiated after the onset of severe trauma-hemorrhage and aiming at reducing the production of pro-inflammatory cytokine are more relevant to prevent the events leading to MODS.

During pregnancy, the maternal immune system tolerates the fetus by reducing the cell-mediated immune response while retaining normal humoral immunity ^{[20]}. Also, clinical symptoms of cell-mediated autoimmune diseases regress in many patients during pregnancy ^{[20]}. The hormone human chorionic gonadotropin (hCG) is mainly secreted by placental syncytiocytotrophoblasts during pregnancy and has been shown to be immunoregulatory.^{[21,22, 23]}. The β-subunit of hCG is degraded by specific proteolytic enzymes ^{[24]}. This can lead to the release of several oligopeptides consisting of four to seven amino acids which, because of their role in regulation of physiological processes, are considered regulatory ^{[25]}. We successfully demonstrated that synthetic oligopeptides can inhibit the acute inflammatory response, disease severity, and mortality in high-dose lipopolysaccharide induced systemic inflammatory response syndrome ^{[26]}. Considering these powerful regulating effects of synthetic oligopeptides on inflammation, we hypothesized that the administration of such regulatory oligopeptides after severe trauma-hemorrhage could inhibit the massive inflammatory response, associated with this condition. To this end, we used LQGV, which is part of the primary structure of loop two of the β-subunit of hCG, and two alanine replacement variants, namely AQGV and LAGV.

In this study we demonstrate that LQGV, AQGV and LAGV, administrated after the induction of hemorrhagic shock in rats, significantly reduced TNF-α and IL-6 plasma levels, which is associated with reduced TNF-α and IL-6 mRNA transcript levels in the liver. This indicates that LQGV, AQGV, LAGV may have therapeutic potential with beneficial effects on systemic inflammation, thereby reducing organ integrity/function, which is associated with severe hemorrhagic shock.

### Materials and Methods

### Animals

Adult male specific pathogen-free Wistar rats *(Harlan CPB, Zeist, The Netherlands),* weighing 350-400g were used. Animals were housed under barrier conditions at 25°C with a twelve-hour light/dark cycle, and were allowed food and water *ad libitum.* The experimental protocol was approved by the Animal Experiment Committee under the Dutch Experiments on Animals Act and adhered to the rules laid down in this national law that serves the implementation of "Guidelines on the protection of experimental animals" by the Council of Europe (1986), Directive 86/609/EC.

*synthetic oligopeptides:* The oligopeptides (LQGV, AQGV and LAGV) were synthesized by Ansynth Service B.V. *(Roosendaal, The Netherlands)* and dissolved in 0.9% NaCl at a concentration of 10 mg/ml.

*Surgical procedures:* Rats were food deprived overnight before the experiment, but were allowed water *ad libitum.* Rats were anesthetized using a mixture of N₂O/O₂ isoflurane *(Pharmachemie B.V., Haarlem, the Netherlands).* Body temperature was continuously maintained at 37.5°C by placing the rats on a thermo controlled 'half-pipe' *(UNO, Rotterdam, The Netherlands).* Endotracheal intubation was performed, and rats were ventilated at 60 breaths per minute with a mixture of N₂O/O₂ 2% isoflurane. Polyethylene tubes *(PE-50, Becton Dickinson; St. Michielsgestel, The Netherlands)* were flushed with heparin and placed via the right carotid artery in the aorta and in the right internal jugular vein. The rats received no heparin before or during the experiment.

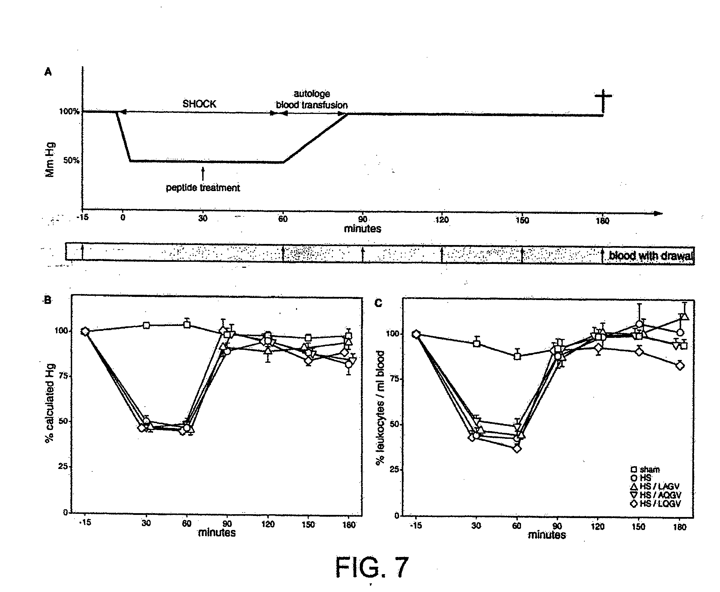

*Experimental procedures:* After an acclimatisation period of 15 minutes, the rats were randomised into five different groups: 1) sham, 2) hemorrhagic shock (HS), 3) hemorrhagic shock with LQGV treatment (HS/LQGV), 4) hemorrhagic shock with AQGV treatment (HS/AQGV) and 5) hemorrhagic shock with LAGV treatment (HS/LAGV). Hemorrhagic shock was induced by blood withdrawal, reducing the circulating blood volume until a mean arterial pressure (MAP) of 50% of normal mmHg was reached. This level of hypotension was maintained for 60 minutes. After 30 minutes, rats received either a single bolus injection of 10 mg/kg LQGV, AQGV, LAGV or 0,9% NaCl solution. The peptides and dosage were based on previous studies, in which we performed dose-escalation experiments (manuscript in preparation). Sixty minutes after induction of hemorrhagic shock, rats were resuscitated by autologous blood transfusion over a period of 30 minutes and monitored for another 120 minutes after which they were sacrificed (Fig.7A). Sham animals underwent the same surgical procedure as the hemorrhagic shock animals, but without performing hemorrhage and administration of peptides.

*Plasma collection and storage:* Arterial blood was obtained 15 minutes before and 30, 60, 90, 120, 150 and 180 minutes after onset of hemorrhage (Fig. 7A). After blood withdrawal, leukocyte numbers were determined using a coulter counter *(Beckman Coulter, Mijdrecht, the Netherlands)* and corrected for the hematocryte. Approximatly, 0,3 ml of blood was placed into mini collect tubes *(Greiner, Bio-one, Alphen a*/*d Rijn, the Netherlands),* plasma was obtained by centrifugation (1500 r.p.m.; 5 minutes), immediately frozen, and stored at -80°C, until assayed.

*Measurements of Mean arterial pressure:* During the experiments, mean arterial pressure (MAP) was continuously measured using transducers *(Becton Dickinson)* that were connected in line to an electronic recorder *(Hewlett Packard, 78354-A, Germany).*

*Tissue collection and storage:* Liver, lungs, ileum and sigmoid were surgically removed at the end of the experiment, snap-frozen, and stored at -80°C, until assayed.

*Measurement of cytokines:* TNF-α and IL-6 plasma levels were determined by ELISA *(R&D Systems Europe Ltd, Abingdon, UK),* according to the manufacturer's instructions.

*Evaluation of mRNA levels by real-time quantitative (RQ)-PCR:* RNA was isolated using a *QIAGEN* kit *(QIAGEN, Hidden, Germany)*, according to the manufacturer's instructions. TNF-α, IL-6 and ICAM-1 transcripts were determined by RQ-PCR using an Applied Biosystems 7700 PCR machine (Foster City, CA, USA) as described previously ^{[27]}. TNF-α, IL-6 and ICAM-1 expression was quantified by normalization against GAPDH. Primer probe combinations used are listed in Table 1.

*Statistical analysis:* Statistical analysis was performed using SPSS version 11 software (SPSS Inc., Chicago,Ill). Inter group differences were analysed with Kruskal-Wallis statistical test. If Kruskal-Wallis statistical testing resulted in a p < 0.05, a Dunn's Multiple Comparison test was performed and p < 0.05 was considered statistically significant.

### RESULTS

*Induction of hemorrhagic shock:* Lowering the MAP to 50% of normal induced hemorrhagic shock, which was successfully maintained for 60 minutes in all four experimental groups (Fig. 7B). No change in MAP was observed in sham treated rats (Fig. 7B). A decrease in the percentage of blood leukocytes was observed in all four experimental groups after blood withdrawal (Fig. 7C). Sixty minutes after hemorrhagic shock, rats were resuscitated with there own blood to induce organ reperfusion, which was associated with a normalization of leukocyte level (Fig. 7C).

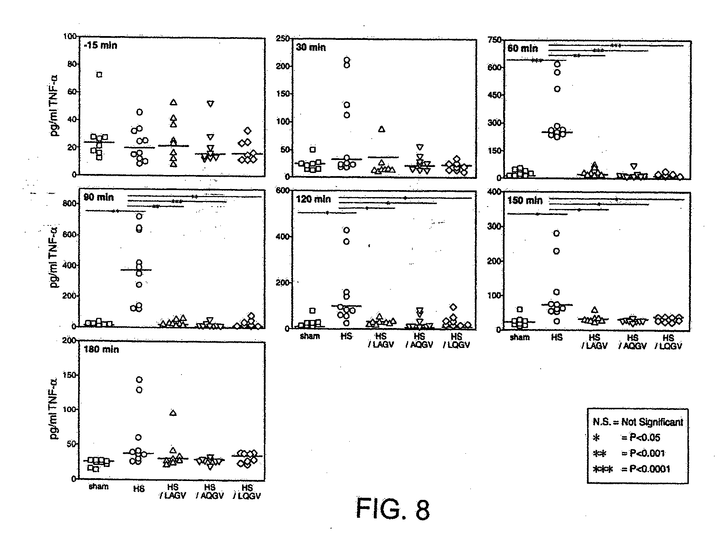

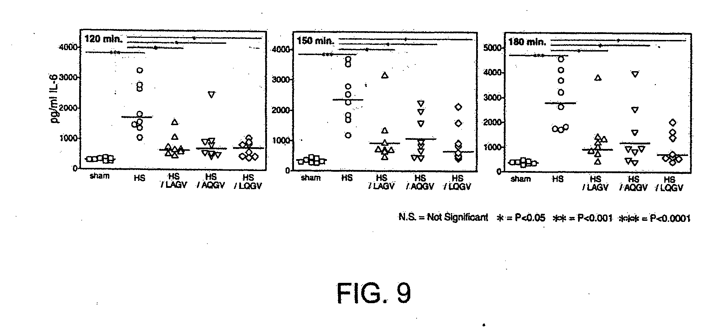

*Oligopeptide treatment reduces pro-inflammatory cytokine plasma levels:* The therapeutic capacity of three synthetic oligopeptides (LQGV, AQGV, LAGV) related to the primary structure of loop two of the β-subunit of hCG was evaluated in a rat hemorrhagic shock model. Before induction of hemorrhage, TNF-α plasma levels were comparable in all five groups (∼15-24 pg/ml) (Fig. 8). In the HS group, TNF-α levels started to increase thirty minutes after induction of hemorrhagic shock and were significantly increased after sixty minutes, as compared to the sham group (264 pg/ml vs 24 pg/ml, respectively; p < 0.01). TNF-α levels reached a maximum of 374 pg/ml after 90 minutes in the HS group, after which levels declined again but always remaining increased compared to the sham group (Fig. 8). In contrast, none of the oligopeptide-treated HS groups (HS/LQGV, HS/AQGV, HS/LAGV) showed an increase in plasma TNF-α levels during the experiment (Fig. 8). IL-6 levels are known to increase at a later time-point than TNF-α after severe hemorrhagic shock ^{[11, 12]}. Therefore, we determined IL-6 levels in blood samples collected 120, 150 and 180 minutes after the onset of hemorrhagic shock. In the HS group, IL-6 plasma levels were significantly increased as compared to sham group at 120 minutes (1704 pg/ml vs 338 pg/ml, respectively; p < 0.001), at 150 minutes (2406 pg/ml vs 316 pg/ml, respectively; p < 0.001) and at 180 minutes (2932 pg/ml vs 369 pg/ml, respectively; p < 0.001) (Fig. 9). Although IL-6 levels tended to increase a little in the HS/oligopeptide treated rats as compared to sham treated rats, this never reached significance. Treatment with oligopeptides after hemorrhagic shock (HS/LQGV, HS/AQGV, HS/LAGV) resulted in a significant reduction of IL-6 plasma levels as compared to the non-treated hemorrhagic shock group (HS) (Fig. 9). These data demonstrate that treatment with a single dose of either LAGV, AQGV, or LAGV after induction of hemorrhagic shock results in a significant reduction of TNF-α and IL-6 plasma levels.

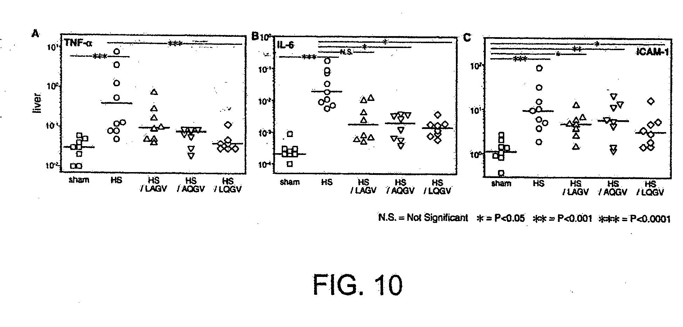

*Oligopeptide treatment reduces TNF-α and IL-6 but not ICAM-1 mRNA levels in the liver:* Because oligopeptide treatment clearly decreased the TNF-α and IL-6 plasma levels, we analysed mRNA levels in liver, lungs, ileum and sigmoid tissues at 180 minutes after the onset of hemorrhagic shock. In the liver, TIVF-α transcripts were significantly increased in the HS group as compared to the sham group. Oligopeptide treatment was associated with decreased TNF-α transcripts in the liver as compared to non-treated HS rats with only HS/LQGV showing a significant reduction as compared to HS (p < 0.01; Fig. 10A).

In the HS group, IL-6 transcripts in the liver were increased ∼83 times as compared to the sham group (p < 0.001; Fig. 10B). None of the oligopeptide treated groups showed an increase in IL-6 mRNA as compared to the sham treated group. LQGV and AQGV treatment resulted in a significant reduction in IL-6 mRNA transcripts as compared to the HS group (p < 0.05; Fig. 10B).

ICAM-1 transcript levels in the liver were significantly increased in the HS group as compared to the sham group (Fig. 10C). Oligopeptide treatment during hemorrhagic shock (HS/LQGV, HS/AQGV, HS/LAGV) did not affect the ICAM-1 transcript levels in the liver (Fig. 10C). In lungs, ileum and sigmoid tissue no significant differences could be detected between the various groups for TNF-α, IL-6 and ICAM-1 (data not shown). These data indicate that oligopeptide treatment following hemorrhagic shock decreases pro-inflammatory cytokine transcript levels in the liver but does not reduce CAM-1 transcript levels.

### DISCUSSION

In this study we used a rat model of hemorrhagic shock and demonstrated that administration of synthetic oligopeptides (LQGV, AQGV, LAGV) 30 minutes after shock induction, efficiently reduces the pro-inflammatory cytokine levels associated with this condition. Our data demonstrate this to be a likely consequence of reduced expression of pro-inflammatory cytokine mRNA transcript levels in the liver.

Hemorrhagic shock is associated with an early adherence of leukocytes to the vascular endothelium as a result of a decreased blood volume ^{[28]}. In our model a decrease in the percentage of leukocytes was detected in all four experimental groups after blood withdrawal. This indicates that all experimental groups experienced hemorrhagic induced shock. Resuscitation resulted in an increase of the percentages of leukocytes in the experimental groups.

Hemorrhagic shock followed by resuscitation induces a severe inflammatory response, which is characterized by an exaggerated production of early pro-inflammatory cytokines, such as TNF-α, IL1β, and subsequently IL-6 ^{[10, 11, 12]}. TNF-α is a key mediator of the innate immune system that is crucial for the generation of a local protective immune response against infectious or non-infectious agents ^{[9]}. However, uncontrolled massive TNF-α production is lethal, as it spreads via the bloodstream into other organs thereby inducing tissue damage and promoting the production of secondary pro-inflammatory mediators, such as IL6 ^{[10, 11]}.

Despite improvement in treatment strategies, trauma-hemorrhage patients may still develop severe inflammatory response that leads too MODS and finally death. Experimental treatment strategies aimed at neutralizing bioactive cytokines, such as monoclonal antibodies against TNF-α, have been successfully applied in several inflammatory disorders, including Crohn's disease and Rheumatoid Arthritis ^{[29, 30]}. However, clinical studies using monoclonal antibodies against TNF-α showed no clinical effect in trauma-patients ^{[31]}. It has been suggested that TNF-α neutralising antibodies causes the accumulation of a large pool of TNF-α/anti-TNF-α pool, which act as a slow release reservoir that may lead to increased constant active TNF-α ^{[32]}. Therefore, aiming at therapies that decrease the production of TNF-α and IL-6 may be more beneficial in limiting tissue damage and mortality rates in trauma-hemorrhage patients than neutralization of already produced cytokines.

In hemorrhagic shock, TNF-α is secreted within minutes after cellular stimulation, while production stops after three hours, and TNF-α plasma levels become almost undetectable ^{[9]}. We demonstrate that regulatory oligopeptides (LQGV, AQGV, LAGV), administered 30 minutes after the induction of hemorrhagic shock, significantly reduced TNF-α and IL-6 plasma levels. Whether the effect on IL-6 production is direct, or indirect due to reduced TNF-α plasma levels cannot be concluded from our data. Nevertheless, establishing a reduction of IL-6 is of clinical importance, because high IL-6 plasma levels correlate with poor outcome and decreased survival in patients with severe trauma and infection ^{[33, 34]}. Cells within the liver, are considered as the main producers of pro-inflammatory cytokines during hemorrhagic shock ^{[17]}. TNF-α and IL-6 transcript levels were significantly increased in the livers of the HS group. LQGV, AQGV or LAGV treatment was associated with a reduction in TNF-α and IL-6 liver transcripts, which may be indicative of decreased transcriptional activation. Another important characteristic of endothelial cells and hepatocytes during hemorrhagic shock is increased expression of the adhesion molecule ICAM-1 ^{[7, 8]}. Our study confirms the increased ICAM-1 expression in the liver after hemorrhagic shock. However, LQGV, AQGV, or LAGV treatment did not result in reduced ICAM-1 expression. This could be due to the inability of oligopeptides to interfere with induction of ICAM-1 transcription. In lungs, ileum and sigmoid, we detected no effect of hemorrhagic shock on the induction of TNF-α, IL-6 and ICAM-1 transcripts. This confirms that the liver is the first organ in which the inflammatory response is initiated after hemorrhagic shock and fluid resuscitation ^{[15, 31, 32]}.

In literature, it is well described that hCG can regulate the immune system, because of it's putative role in preventing the rejection of the fetal allograft during pregnancy ^{[35, 36]}. Human CG exerts its function by binding to specific membrane-bound receptors, which activate second messengers ^{[37]}. The oligopeptides are expected to cross cell membranes without requiring membrane-bound receptors ^{[38]} and exert their effects intracellularly. This study and ongoing studies in our laboratory demonstrate that these oligopeptides have a distinct regulating effect on the expression of genes involved in inflammatory pathways and immunity. Nevertheless, investigation on the mechanism of action of hCG-related peptides regulate gene expression are necessary.

Recently a study was published in which the dipeptide AG inhibited the mRNA expression of pro-inflammatory cytokines in the liver after hemorrhagic shock ^{[39]}. However, a very high peptide dose of AG was required (150mg/kg), where we observed clear effects using 10mg/kg in our study. Nevertheless, this and our study indicate that the use of specific oligopeptides can be considered as therapeutic agents for treatment of the inflammatory response after severe trauma.

In summary, a single administration of a synthetic oligopeptide (LQGV, AQGV, LAGV) after the induction of severe trauma-hemorrhage reduces the subsequent pro-inflammatory response. These data suggest that these oligopeptides have therapeutic potential, in minimizing the late life threatening tissue- and organ-damage that is associated with severe trauma-hemorrhage.

### REFFERENCES

1. Vincent JL., Carlet, J., Opal, S.M., eds., The sepsis text, 2002
2. Cohen J. The immunopathogenesis of sepsis. Nature 2002; 420:885-891
3. Osborn TM, Tracy JK, Dunne JR, Pasquale M, Napolitano LM. Epidemiology of sepsis in patients with traumatic injury. Crit Care Med. 2004; 32(11):2234-40
4. DS, Lefering R, Wade CE..Impact of hemorrhage on trauma outcome: an overview of epidemiology, clinical presentations, and therapeutic considerations. J Trauma, 2006, 60(6):83-11*.*
5. Muylle L. The role of cytokines in blood transfusion reactions. Blood Rev 1995; 9:77-83*.*
6. Matsutani T, Kang SC, Miyashita M, Sasajima K, Choudhry MA, Bland KI, et al. Liver cytokine production and ICAM-1 expression following bone fracture, tissue trauma, and hemorrhage in middle-aged mice. Am J Physiol Gastrointest Liver Physiol 2007; 292:268-74
7. Rothoerl RD, Schebesch KM, Kubitza M, Woertgen C, Brawanski A, Pina AL. ICAM-1 and VCAM-1 expression following aneurysmal subarachnoid hemorrhage and their possible role in the pathophysiology of subsequent ischemic deficits. Cerebrovasc Dis 2006; 22:143-9
8. Sun LL, Ruff P, Austin B, Deb S, Martin B, Burris D, et al. Early up-regulation of intercellular adhesion molecule-1 and vascular cell adhesion molecule-1 expression in rats with hemorrhagic shock and resuscitation. Shock 1999; 11:416-22*.*
9. Ferguson KL, Taheri P, Rodriguez J, Tonapi V, Cardellio A, Dechert R.Tumor necrosis factor activity increases in the early response to trauma. Acad Emerg Med. 1997; 4(11):1035-40*.*
10. Mainous MR, Ertel W, Chaudry IH, Deitch EA. The gut: a cytokinegenerating organ in systemic inflammation? Shock 1995; 4:193-9
11. Yang R, Han X, Uchiyama T, Watkins SK, Yaguchi A, Delude RL, Fink MP, IL-6 is essential for development of gut barrier dysfunction after hemorrhagic shock and resuscitation in mice. Am J Physiol Gastrointest Liver Physiol. 2003;285(3): G621-9
12. Taniguchi T, Koido Y, Aiboshi J, Yamashita T, Suzaki S, Kurokawa A, Change in the ratio of interleukin-6 to interleukin-10 predicts a poor outcome in patients with systemic inflammatory response syndrome. Crit Care Med 22.1999; 1262-1264*.*
13. Snyder EL. The role of cytokines and adhesive molecules in febrile non-hemolytic transfusion reactions. Immunol Invest 1995; 24:333-9.
14. Winkelstein A, Kiss JE. Immunohematologic disorders. Jama 1997; 278:1982-92*.*
15. Davenport R. Cytokines and erythrocyte incompatibility. Curr Opin Hematol 1994; 1:452-6
16. Davenport RD. The role of cytokines in hemolytic transfusion reactions. Immunol Invest 1995; 24:319-31*.*
17. Zhu XL, Zellweger R, Zhu XH, Ayala A, Chaudry IH, Cytokine gene expression in splenic macrophages and Kupffer cells following haemorrhage, Cytokine. 1995; 7(1):8-14*.*
18. fong Y, Tracey KJ,. Moldawer LL, Hesse DG, Manogue KB, Kenney JS., Lee AT, Kuo GC, Allison AC, Lowry SF, Cermani CA, Antibodies to cachectin/tumor necrosis factor reduce interleukin 1 beta and interleukin 6 appearance during lethal bacteremia, J.Exp. Med., 1989; 1;170(5):1627-33
19. Robinson MK, Rounds JD, Hong RW, Jacobs DO, Wilmore DW. Glutathione deficiency increases organ dysfunction after hemorrhagic shock. Surgery 1992; 112:140-7*; discussion 8-9*
20. Sacks G, Sargent I, Redman C, An innate view of human pregnancy. Immunol Today, 1999, 20(3):114-8
21. Muyan M. and Boime I., Secretion of chorionic gondatrophin from human trophoblast. Placenta, 1997; 19(4):237-41
22. Alexander H, Zimmermann G, Lehmann M, Pfeiffer R, Schone E, Leiblein S, Ziegert, M, HCG secretion by peripheral mononuclear cells during pregnancy, Domest Anim Endocrinol.;1998; 15(5): 377-87
23. Khan NA, Khan A, Savelkoul HF, Benner R, Inhibition of diabetes in NOD mice by human pregnancy factor. Hum Immunol., 2001, 62(12):1315-23*.*
24. Cole LA, The deactivation of hCG by nicking and dissociation. J. Clin Endocrinical metab.,1993; 76(3)704-10
25. Benner R, Khan NA. Dissection of systems, cell populations and molecules. Scand J Immunol 2005; 62 Suppl 1: 62-6*.*
26. Khan NA, Khan A, Savdlkdul HF, Benner R. Inhibition of septic shock in mice by an oligopeptide from the beta-chain of human chorionic gonadotrophin hormone. Hum Immunol 2002; 63:1-7
27. Beillard E, Pallisgaard N, van der Velden VH, Bi W, Dee R, van der Schoot E, Delabesse E, Macintyre E, Gottardi E, Saglio G, Watzinger F, Lion T, van Dongen JJ, Hokland P, Gabert J.Evaluation of candidate control genes for diagnosis and residual disease detection in leukemic patients using 'real-time' quantitative reverse-transcriptase polymerase chain reaction (RQ-PCR) - a Europe against cancer program. Leukemia. 2003; 17.2474-2486*.*
28. Childs EW, Wood JG, Smalley DM, Hunter FA, Cheung LY. Leukocyte adherence and sequestration following hemorrhagic shock and total ischemia in rats. Shock. 1999;11(4):248-52
29.Suenaert P, Bulteel V, Lemmens L, Noman M, Geypens B, Van Assche G, Geboes K, Ceuppens JL, Rutgeerts P. Anti-tumor necrosis factor treatment restores the gut barrier in Crohn's disease, Am J Gastroenterol. 2002 ;97(8):2000-4*.*
30. Olsen NJ, Stein CM. New drugs for rheumatoid arthritis. N Engl J Med. 2004 350(21): 2167- 79
31. Kox WJ, Volk T, Kox SN, Volk HD, Immunomodulatory therapies in sepsis. Intensive Care Med., 2000, 26 (1)5124-8
32. Jit M, Henderson B, Stevens M, Seymour RM, TNF-alpha neutralization in cytokine-driven diseases: a mathematical model to account for therapeutic success in rheumatoid arthritis but therapeutic failure in systemic inflammatory response syndrome. Rheumatology, 2005 Mar;44(3):323-31*.*
33. Yang R, Han X, Uchiyama T, Watkins SK, Yaguchi A, Delude RL, Fink MP, IL-6 is essential for development of gut barrier dysfunction after hemorrhagic shock and resuscitation in mice. Am J Physiol Gastrointest Liver Physiol. 2003;285(3): G621-9
34. Taniguchi T, Koido Y, Aiboshi J, Yamashita T, Suzaki S, Kurokawa A, Change in the ratio of interleukin-6 to interleukin-10 predicts a poor outcome in patients with systemic inflammatory response syndrome. Crit Care Med 27:1999; 1262-1264
35. Stites DP, Pavia CS, Clemens LE, Kuhn RW, Siiteri PK, Immunologic regulation in pregnancy, Arthritis Rheum. 1979 22(11):1300-7*.*
*36.* Saito S, Cytokine network at the feto-maternal interface, J Reprod Immunol., 2000, 47(2):87-103*.*
37. Puett D, Li Y, DeMars G, Angelova K, Fanelli F, A functional transmembrane complex: the luteinizing hormone receptor with bound ligand and G protein, Mol Cell Endocrinol. 2007 2;260-262*:126-36.*
38.Bai JPF, Subramanian P, Mosberg HI and Amidon GL, Structural requirements for the intestinal mucosal-cell peptide transport: the need for N-terminal α-amino group, 1991, Pharm Res 8: 593-599*.*
39.Yang R, Tan X, Thomas AM, Steppacher R, Qureshi N, Morrison DC, Van Way CW 3rd. Alanine-glutamine dipeptide (AGD) inhibits expression of inflammation-related genes in hemorrhagic shock. J Parenter Enteral Nutr. 2007;31(1):32-6*.*

**Table 1. Primers and probes used to determine transcription levels for TNF-α, IL-6, IC-AM-1 and GAPDH..**

| mRNA transc ript | Forward primer | Reverse primer | Probe | Produc t size (bp.) |
|---|---|---|---|---|
| TNF-α | F-RAT-TNFa-EMC TGCCTCAGCCTCTTCTC ATTC | R-RAT-TNFa-EMC GGTCTGGGCCATGGA ACTG | T-RAT-TNFa-EMC FAM- CCACCACGCTCTTCTGTCTACTGAACTTCG- TAMRA | 138 |
| IL-6 | F-RAT-IL6-EMC2 CAGAGGATACCACCCAC AACAGA | R-RAT-IL6-EMC2 GCCATTGCACAACTC TTTTCTCA | T-RAT-IL6-EMC FAM- ACCACTTCACAAGTCGGAGGCTTAATTACAT ATGTTCT-TAMRA | 106 |
| ICAM-1 | F-RAT-ICAM1-EMC TGGGGAAGACAGCAGA CCA | R-RAT-ICAM1-EMC CAGCGCAGGATGAG GTTCT | T-RAT-ICAM1-EMC FAM-CACCACGCAGTCCTCGGCTTCTG- TAMRA | 153 |
| GAPD H | F-RAT-GAPDH-EMC GGATACCACCCACAACA GACCAGTA | R-RAT-GAPDH-EMC TTTTGGCCCCACCCT TCA | T-RAT-GAPDH-EMC FAM- ACTCCACGACATACTCAGCACCAGCATCA- TAMRA | 131 |

## Claims

1. Use of at least one isolated or synthetic tetrapeptide selected from the group consisting of LQGV, AQGV and LAGV and functional derivatives thereof wherein an L-amino acid residue is substituted with a D-amino acid, or a pharmaceutically acceptable acid- or base addition salt thereof, for the production of a pharmaceutical composition for the treatment of a subject suffering from or believed to be suffering from trauma-hemorrhage.

2. Use according to claim 1, wherein said peptide is selected from the group consisting of LQGV, AQGV and LAGV.

3. Use according to claim 1 or 2, comprising the use of a mixture of (i) a tetrapeptide selected from the group consisting of LQGV, AQGV and LAGV and (i) a further peptide capable of reducing pro-inflammatory cytokine levels in an animal model of trauma-hemorrhage or hemorrhagic shock.

4. Use according to any one of claims 1 to 3, wherein said tetrapeptide is LQGV.

5. Use according to any one of claims 1 to 3, wherein said tetrapeptide is AQGV.

6. Use according to any one of claims 1 to 3, wherein said tetrapeptide is LAGV

7. Use according to anyone of claims 1 to 6, wherein said treatment of trauma-hemorrhage also comprises providing said subject with blood or blood products such as red blood cells (RBC's), platelets, plasma, or combinations thereof.

## Patentansprüche

1. Verwendung von mindestens einem isolierten oder synthetischen Tetrapeptid, ausgewählt aus der Gruppe, bestehend aus LQGV, AQGV und LAGV und funktionellen Derivaten davon, wobei ein L-Aminosäurerest durch eine D-Aminosäure oder ein pharmazeutisch akzeptables Säure- oder Basezusatzsalz davon substituiert wird, zur Herstellung einer pharmazeutischen Zusammensetzung für die Behandlung eines Patienten, der an traumatischer Hämorrhagie leidet oder vermutlich daran leidet.

2. Verwendung nach Anspruch 1, wobei das Peptid ausgewählt ist aus der Gruppe bestehend aus LQGV, AQGV und LAGV.

3. Verwendung nach Anspruch 1 oder 2, umfassend die Verwendung eines Gemischs aus (i) einem Tetrapeptid, ausgewählt aus der Gruppe, bestehend aus LQGV, AQGV und LAGV, und (ii) einem weiteren Peptid, das in der Lage ist, pro-inflammatorische Cytokin-Spiegel in einem Tiermodell für traumatische Hämorrhagie oder hämorrhagischen Schock zu reduzieren.

4. Verwendung nach einem der Ansprüche 1 bis 3, wobei das Tetrapeptid LQGV ist.

5. Verwendung nach einem der Ansprüche 1 bis 3, wobei das Tetrapeptid AQGV ist.

6. Verwendung nach einem der Ansprüche 1 bis 3, wobei das Tetrapeptid LAGV ist.

7. Verwendung nach einem der Ansprüche 1 bis 6, wobei die Behandlung der traumatischen Hämorrhagie auch umfasst, dass der Patient mit Blut oder Blutprodukten wie roten Blutkörperchen (RBK), Blutplättchen, Plasma oder Kombinationen davon versorgt wird.

## Revendications

1. Utilisation d'au moins un tétrapeptide isolé ou synthétique sélectionné dans le groupe consistant en LQGV, AQGV et LAGV et des dérivés fonctionnels de ceux-ci dans lesquels un résidu d'acide L-aminé est substitué par un acide D-aminé, ou un sel d'addition d'acide ou de base pharmaceutiquement acceptable de ceux-ci, pour la production d'une composition pharmaceutique destinée au traitement d'un sujet souffrant ou pour lequel on pense qu'il souffre d'une hémorragie traumatique.

2. Utilisation selon la revendication 1, dans laquelle ledit peptide est sélectionné dans le groupe consistant en LQGV, AQGV et LAGV.

3. Utilisation selon la revendication 1 ou 2, comprenant l'utilisation d'un mélange de (i) un tétrapeptide sélectionné dans le groupe consistant en LQGV, AQGV et LAGV et (ii) un autre peptide capable de réduire des taux de cytokines pro-inflammatoires chez un modèle animal d'hémorragie traumatique ou de choc hémorragique.

4. Utilisation selon l'une quelconque des revendications 1 à 3, dans laquelle ledit tétrapeptide est LQGV.

5. Utilisation selon l'une quelconque des revendications 1 à 3, dans laquelle ledit tétrapeptide est AQGV.

6. Utilisation selon l'une quelconque des revendications 1 à 3, dans laquelle ledit tétrapeptide est LAGV.

7. Utilisation selon l'une quelconque des revendications 1 à 6, dans laquelle ledit traitement d'une hémorragie traumatique consiste également à fournir audit sujet du sang ou des produits sanguins comme des globules rouges (GR), des plaquettes, du plasma, ou des combinaisons de ceux-ci.
